# EUROPEAN PATENT APPLICATION

(11) **EP 4 831 283 A1**
(43) Date of publication of application: **28.10.2026**
(21) Application number: 24906427.0
(22) Date of filing: 19.12.2024
(51) Int. Cl.: C12N 9/18, C12N 15/55

(54) **MUTANT LYSOPHOSPHOLIPASE**

(30) Priority: 19.12.2023 CN 202311750772; 22.12.2023 CN 202311787389
(71) Applicant: Wilmar (shanghai) Biotechnology Research & Development Center Co., Ltd., Shanghai 200137 (CN); Wilmar International Limited, Singapore 138568 (SG)
(72) Inventor: WU, Wei, Shanghai 200137 (CN); XUAN, Yaoji, Shanghai 200137 (CN); CHUA, Nam-Hai, Wilmar International 138568 (SG); TANG, Yi, Shanghai 200137 (CN); ZHOU, Liyun, Shanghai 200137 (CN); WANG, Shengnan, Shanghai 200137 (CN)
(74) Representative: Hoffmann Eitle
(86) International application number: PCT/CN2024/140463
(87) International publication number: WO 2025/130948

(57) **Abstract**

The present invention relates to a mutant lysophospholipase. The present invention further relates a gene encoding the enzyme, a vector including the gene, and a host cell. In addition, the present invention relates to a use of the enzyme.

## Description

### TECHNICAL FIELD

The present invention relates to a mutant lysophospholipase. The present invention further relates a gene encoding the enzyme, a vector including the gene, and a host cell. In addition, the present invention relates to a use of the enzyme.

### BACKGROUND ART

Two lipases, designated lipase A and lipase B, or lipase 1 and lipase 2, have been reported in Aspergillus niger. The properties of lipase B are relatively special. Zhu Shu-sen cloned and expressed lipase B from Aspergillus niger A733, and found that lipase B had an optimal temperature of 15°C and an optimal pH of 3.5 to 4.0, and could not tolerate a temperature of 40°C or higher. The enzyme was able to hydrolyze substrates of pNPC4 to pNPC18 chain length, and pNPC12 was the optimal substrate. However, lip B had extremely low specific enzyme activity, the specific enzyme activity of purified lip B being only 6.8 U/mg.

Jiangke Yang et al. cloned and expressed lipas 2 from Aspergillus niger CCIC 4009. Although this lipase 2 was highly homologous to the lipase B cloned by Zhu Shu-sen, differing by only two amino acids, the properties were different to some extent. The optimal substrates for the lipase 2 were pNPC8 and pNPC10, and the lipase 2 had an optimal pH of lower than 6.5 and an optimal temperature of 50°C, and could not tolerate a temperature of 40°C or higher.

In summary, the liapse 2 or lipase B derived from Aspergillus niger is hardly practical. Firstly, the enzyme has extremely low specific enzyme activity and a low temperature tolerance, and the applicable optimal substrates for the enzyme are triglycerides of short-chain fatty acids. The enzyme is inferior to the more widely used lipases TL and RML, the latter two lipases having specific enzyme activity of up to 12000 U/mg or 8000 U/mg. TL can withstand a temperature of 60°C for 20 hours without deactivation, and RML can hydrolyze triglycerides of various long-chain fatty acids.

In the earlier patent application CN202110707919.4, the present inventors designed primers using the gene sequence of lipase B of CBS513.88 to clone the LPL (lysophospholipase) gene AN02-LPL from the Aspergillus niger GIM 3.24 (AN02) strain, and found that AN02-LPL had very high phospholipase A1 activity and lysophospholipase activity, and could be used for oil degumming. Without the addition of an alkali, AN02-LPL could reduce the phosphorus content of crude oil to 5 ppm. AN02-LPL can be used for enzymatic degumming, and since alkali addition is not needed, soap formation during degumming can be reduced. In addition, since AN02-LPL has extremely high lysophospholipase activity, AN02-LPL can be used in combination with phospholipase A2 to prepare glycerophosphorylcholine (GPC) using soybean phospholipid as a starting material. GPC has the effect of invigorating the brain and preventing aging, and is used in pharmaceuticals and health-care products. However, when the gene is applied to pre-degummed soybean oil and rice, the degumming effect fails to meet requirements.

There remains a desire in the art for a lysophospholipase having higher specific enzyme activity and stability.

### SUMMARY OF THE INVENTION

On the basis of the previous work, the present inventors developed a novel mutant based on the mutant lysophospholipase developed in the invention CN202110707919.4.

Specifically, the present inventors obtained one mutant D94N of AN02-LPL by a method of site-directed mutagenesis of aspartic acid at position 94 of wild-type lysophospholipase. The mutant D94N had a 77% reduction in the loss of enzyme activity after storage at 4°C for 2 weeks compared to AN02-LPL.

In addition, the present inventors mutated the amino acids at positions 84 and 85 to arginine and threonine, respectively, improving the specific enzyme activity for phosphatidic acid. The mutant had better application performance in the degumming of pre-degummed soybean oil than that of the wild-type gene AN02-LPL, and could reduce the phosphorus content to a lower level, meeting the requirements of physical refining.

On the basis of AN02-LPL, the present inventors further mutated lysine at position 111 to histidine, tyrosine at position 147 to leucine, serine at position 84 to arginine, and aspartic acid at position 85 to threonine. The specific enzyme activity of the new mutant HLRT against phosphatidic acid increased from 347 U/mg of AN02-LPL to 835 U/mg at pH 4.3; increased from 863 U/mg of AN02-LPL to 2903 U/mg at pH 4.8; and increased from 0 U/mg of AN02-LPL to 2527 U/mg at pH 5.5.

On the basis of the mutant polypeptide M1 of AN02-LPL (the mutation sites were L86I, G187D, E209K, and A254D based on the mature peptide), the present inventors further mutated serine at position 84 to arginine and aspartic acid at position 85 to threonine. The specific enzyme activity of the new mutant M1RT against phosphatidic acid increased from 347 U/mg of AN02-LPL to 1252 U/mg at pH 4.3; increased from 863 U/mg of AN02-LPL to 2956 U/mg at pH 4.8; and increased from 0 U/mg of AN02-LPL to 2972 U/mg at pH 5.5.

On the basis of the AN02-LPL gene, the present inventors further mutated serine at position 84 to arginine and aspartic acid at position 85 to threonine. The specific enzyme activity of the new mutant WTRT against phosphatidic acid increased from 347 U/mg of AN02-LPL to 984 U/mg at pH 4.3; increased from 863 U/mg of AN02-LPL to 2003 U/mg at pH 4.8; and increased from 0 U/mg of AN02-LPL to 1342 U/mg at pH 5.5.

On the basis of the above WTRT, the present inventors further mutated aspartic acid at position 94 to tyrosine to obtain a new mutant WTRT-Y. The new mutant WTRT-Y could still maintain high specific enzyme activity against phosphatidic acid, and the specific enzyme activity increased from 347 U/mg of AN02-LPL to 961 U/mg at pH 4.3; increased from 863 U/mg of AN02-LPL to 1803 U/mg at pH 4.8; and increased from 0 U/mg of AN02-LPL to 1212 U/mg at pH 5.5. The enzyme activities were substantially equivalent to those of the mutant WTRT (984 U/mg at pH 4.3, 2003 U/mg at pH 4.8, and 1342 U/mg at pH 5.5).

The mutant WTRT showed a decrease in enzyme activity from 29921 U/ml to 20076 U/ml after 2 weeks of storage at 4 degrees, whereas the mutant WTRT-Y showed an increase in protein stability in that the enzyme activity thereof only decreased from 28765 U/ml to 25984 U/ml after 2 weeks of storage at 4 degrees. The loss decreased by 68% compared to that of WTRT.

A strain expressing the mutant WTRT, after being subcultured two times, had enzyme activity of only 37.5% of the non-subcultured strain, whereas a strain expressing the mutant WTRT-Y, after being subcultured two times, had enzyme activity consistent with that of the non-subcultured strain, without the phenomenon of instability.

Specifically, the present invention relates to the following aspects:
In one aspect, the present invention relates to a mutant lysophospholipase, having one or more mutations in the amino acid sequence thereof relative to a wild-type lysophospholipase and having increased specific activity or increased stability.

A person skilled in the art will appreciate that the wild-type lysophospholipase that serves as the basal sequence of the mutant lysophospholipase of the present invention is not limited to SEQ ID NO: 1, but may also include other lysophospholipases known in the art. In particular, the lysophospholipase of the present invention may have, relative to lysophospholipases known in the art, mutations at the amino acid positions corresponding to positions 84 and 85 of SEQ ID NO: 1.

For example, the sequence of a known lysophospholipase (manufactured by Novozymes A/S) serving as the basal sequence for mutation may be:

MHRPLQLWALAALTSLVTAAPAPVLRRDVSSSVLSELDLFAQYSAAAYCSSNIG
SPGTKLTCSVGNCPRVEAADTETLIEFNESSSFGDVTGYIAVDRTNSLLVLAFRGSSTVSN
WEADLDFPLTDASSLCSGCEIHSGFWAAWQTVQASITSTLESAIASYPGYTLVFTGHSYG
AALAAIAATTLRNAGYTIQLYDYGQPRLGNLALAQYITAQTQGANYRVTHTDDIVPKLP
PELFGYHHFSPEYWITSGDNVTVTTSDVQVVTGIDSTAGNDGTLLDSTSAHDWYIVYID
GCD (SEQ ID NO: 8).

Since SEQ ID NO: 8 has 27 more amino acids than SEQ ID NO: 1 at the N terminus, a person skilled in the art will appreciate that the amino acid positions corresponding to positions 84 and 85 of SEQ ID NO: 1 refer to positions 111 and 112 of SEQ ID NO: 8. Thus, the present invention also relates to a lysophospholipase having mutations at positions 111 and 112 of SEQ ID NO: 8.

As another example, a known lysophospholipase as the basal sequence for mutation is SEQ ID NO: 1 in CN2019800303753.

In one aspect, the present invention relates to a lysophospholipase, the amino acid sequence thereof having a mutation or mutations at one or more positions selected from positions 84, 85, and 94 corresponding to SEQ ID NO: 1.

In one embodiment, the amino acid sequence of the lysophospholipase has a mutation at position 94 relative to SEQ ID NO: 1. In one embodiment, the mutation is mutation of aspartic acid to asparagine.

In one aspect, the present invention relates to a lysophospholipase, the amino acid sequence thereof having mutations at positions 84 and 85 relative to SEQ ID NO: 1. In one embodiment, the mutations are mutation of serine at position 84 to arginine and mutation of aspartic acid at position 85 to threonine. In one embodiment, the lysophospholipase further comprises an additional mutation or additional mutations at one or more positions selected from positions 111, 147, 86, 187, 209, and 254. In one embodiment, the additional mutations are mutations at positions 111 and 147. In one embodiment, the additional mutations are mutations at positions 86, 187, 209, and 254. In one embodiment, the additional mutations are mutation of lysine at position 111 to histidine and mutation of tyrosine at position 147 to leucine. In one embodiment, the additional mutations are mutation of leucine at position 86 to isoleucine, mutation of glycine at position 187 to aspartic acid, mutation of glutamic acid at position 209 to lysine, and mutation of alanine at position 254 to aspartic acid.

In one embodiment, the amino acid sequence of the lysophospholipase of the present invention has mutations at positions corresponding to positions 84, 85, and 94 of SEQ ID NO: 1. In one embodiment, the mutations are mutation of serine at position 84 to arginine, mutation of aspartic acid at position 85 to threonine, and mutation of aspartic acid at position 94 to tyrosine.

In one embodiment, the amino acid sequence of the lysophospholipase is as shown in SEQ ID NO: 2, 3, 5, 6, or 7.

In another aspect, the present invention relates to a nucleic acid molecule, the nucleic acid molecule being selected from: (a) a nucleotide sequence encoding the above lysophospholipase; and (b) a nucleotide sequence complementary to the nucleotide sequence according to (a), wherein the complementarity may be partial or complete.

A skilled person will appreciate that, due to the degeneracy of the genetic code, a plurality of different nucleotide sequences may encode the same enzyme. In addition, it will be appreciated that a skilled person is able to use routine techniques to make nucleotide substitutions that do not affect the enzymatic activity encoded by the nucleotide sequence of the present invention, such that the codon bias of any particular host organism used for expressing the enzyme of the present invention may be reflected.

The present invention further provides a vector comprising the nucleic acid molecule, and a host cell comprising the nucleic acid molecule or the vector.

By "vector" it is meant an extrachromosomal element that normally carries a gene that is not part of the central metabolism of a cell, and is often in the form of a circular double-stranded DNA molecule. Such an element may be an autonomously replicating sequence, a genome integration sequence, a phage or nucleotide sequence, or a linear or circular single-stranded or double-stranded DNA or RNA obtained from any source, wherein a number of nucleotide sequences have been joined or recombined into a construct capable of introducing a promoter fragment and a DNA sequence for a selected gene product, along with an appropriate 3' untranslated sequence, into a cell.

The gene and the gene product encoding the lysophospholipase of the present invention may be expressed in a heterologous host cell, such as a bacterial cell, a fungal cell, e.g., a yeast cell, a mammalian cell, an insect cell, and a plant cell. The heterologous host cell used to express the nucleic acid molecule of the present invention may be a microbial host that is present in a fungal or bacterial family and grows over wide ranges of temperature, pH value, and solvent tolerance. For example, it is contemplated that any bacterium, yeast, and filamentous fungus may be a suitable host for expressing the nucleic acid molecule of the present invention. Examples of host strains include, but are not limited to, bacterial, fungal, or yeast species, such as species of Pichia, Aspergillus, Trichoderma, Saccharomyces, Phaffia, Kluyveromyces, Yarrowia, Candida, Hansenula, Salmonella, Bacillus, Acinetobacter, Zymomonas, Agrobacterium, Erythrobacter, Chlorobium, Chromatium, Flavobacterium, Cytophaga, Rhodobacter, Rhodococcus, Streptomyces, Brevibacterium, Corynebacteria, Mycobacterium, Deinococcus, Escherichia, Erwinia, Pantoea, Pseudomonas, Sphingomonas, Methylomonas, Methylobacter, Methylococcus, Methylosinus, Methylomicrobium, Methylocystis, Alcaligenes, Synechocystis, Synechococcus, Anabaena, Thiobacillus, Methanobacterium, Klebsiella, and Myxococcus. In one embodiment, the host cell is a fungal cell. In one embodiment, the host cell is a Pichia pastoris or Aspergillus niger cell.

Vectors useful for transforming the above-mentioned host cells are well known in the art. Typically, a vector comprises a sequence that directs the transcription and translation of a relevant gene, a selectable marker, and a sequence that allows for autonomous replication or chromosomal integration. A suitable vector comprises a 5' region of the gene that contains control for transcription initiation and a 3' region of the DNA fragment that controls transcription termination.

In one aspect, the present invention further relates to a method for producing a lysophospholipase, the method comprising expressing a nucleic acid molecule encoding the lysophospholipase of the present invention in a host cell, and recovering a resulting polypeptide.

Various culture methods may be applied to prepare the enzyme of the present invention. For example, large-scale production of a particular gene product from a recombinant microbial host may be carried out by batch, fed-batch, and continuous culture methods.

Batch and fed-batch culture methods are common and well known in the art, and examples can be found in the following documents: Thomas D. Brock in Biotechnology: A Textbook of Industrial Microbiology, Second Edition, Sinauer Associates, Inc., Sunderland, Mass. (1989)), and Deshpande, Mukund V., (Appl. Biochem. Biotechnol., 36:227-234 (1992).

Commercial production of the enzyme of the present invention may also be carried out by continuous culture. Continuous culture is an open system in which a set medium is continuously added to a bioreactor and, simultaneously, an equal amount of conditioned medium is removed for processing. Continuous culture generally maintains cells at a constant high liquid phase density where the cells are primarily in a logarithmic growth phase. Alternatively, continuous culture may be carried out using immobilized cells, whereby carbon and nutrients are continuously added, and a valuable product, a by-product, or waste is continuously removed from the cell mass. Cell immobilization may be carried out using a wide range of solid supports, the solid supports consisting of a natural and/or synthetic material.

Recovery of the desired enzyme from batch fermentation, fed-batch fermentation, or continuous culture may be accomplished by any method known to a person skilled in the art. For example, when the enzyme is intracellularly produced, a cell slurry is separated from the culture medium by centrifugation or membrane filtration and optionally washed with water or an aqueous buffer of a desired pH, and then the cell slurry in the aqueous buffer of the desired pH is suspended and homogenized to produce a cell extract containing the desired enzyme.

The present invention further relates to a composition comprising a fermentation broth, a fermentation supernatant, and/or a fermentation concentrate of the lysophospholipase of the present invention or the host cell of the present invention. The enzyme composition of the present invention may be in any form suitable for use, for example, a crude fermentation broth from which the cells have or have not been removed, a cell lysate containing or not containing cell debris, a semi-purified or purified enzyme composition, or the host cells, as a source of the enzyme. The enzyme composition may be a dry powder or granule, a non-dusting granule, a liquid, a stabilized liquid, or a stabilized protected enzyme. The liquid enzyme composition may be stabilized according to established processes, for example by the addition of stabilizers such as sugars, sugar alcohols, or other polyols, and/or lactic acid or other organic acids.

The present invention further relates to a fermentation broth, a fermentation supernatant, or a fermentation concentrate of the host cell of the present invention.

The present invention further relates to a use of the lysophospholipase of the present invention in oil degumming. When the lysophospholipase of the present invention is used for oil degumming, specific enzyme activity is significantly improved and production costs are reduced, compared with the lysophospholipase of the prior art.

### BRIEF DESCRIPTION OF THE DRAWINGS

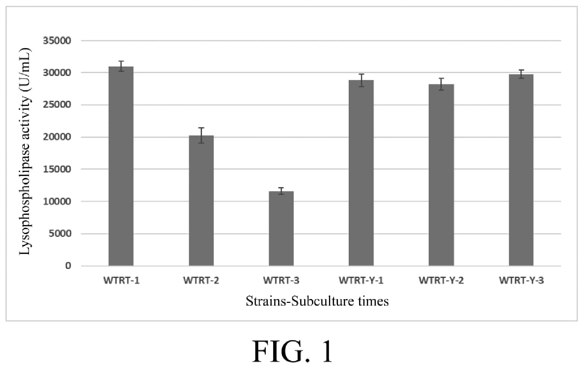

FIG. 1 shows changes in the enzyme activity of mutants WTRT and WTRT-Y after pAOP-WTRT and pAOP-WTRT-Y strains were subcultured two times on PDA solid medium plates.

### DETAILED DESCRIPTION OF THE PREFERRED EMBODIMENTS

### Experimental materials

### 1. Experimental strains and plasmids

Strains: Pichia pastoris GS115 was purchased from Invitrogen, C18100; Aspergillus niger AN19 was purchased from the China Center of Industrial Culture Collection, accession number: Aspergillus niger CCIC2243.

SphI and HindIII restriction enzymes were purchased from NEB.

Lecithin (purity 98%) was purchased from Aladdin.

A NEFA kit was purchased from Wako Pure Chemical Industries, Ltd., Japan.

A Bradford kit was purchased from Sangon (Shanghai) Bioengineering Co., Ltd. Plasmids: A pAOP-Eno plasmid was constructed by our laboratory, as described in detail below. pAO815 was purchased from Invitrogen, K1750-01.

A pAOP-Eno vector was constructed by the present inventors by referring to the method in Molecular Cloning: A Laboratory Manual (3rd Edition, New York, Cold Spring Harbor Laboratory Press, 1989). The specific process is as follows:
An RML gene (NCBI accession number: A34959), carrying an Aspergillus oryzae α-amylase signal peptide (NCBI accession number: XM_001821384.2, 1-63 bp sequence)) obtained from whole gene synthesis by Sangon Biotech (Shanghai) Co., Ltd. was inserted, using SphI and HindIII restriction sites, into an expression cassette including an Aspergillus oryzae enolase promoter (NCBI sequence number: D63941.1, 215-734 bp; containing 12 copies of an enhancer sequence) and an Aspergillus niger glucoamylase terminator (NCBI sequence number: AF214480.1, the terminator sequence portion). The entire expression cassette was inserted, using BglII and XhoI, into a multiple cloning site of cloning vector pSP72. Finally, a PyrG expression gene (NCBI accession number: AB017705.1) derived from Aspergillus oryzae was inserted into a vector using an XhoI restriction site, thereby constructing the RML gene expression vector pAOP-Eno.

### 2. Culture media and solutions

Aspergillus niger screening plate: 1.34% yeast nitrogen base (YNB) containing ammonium sulfate and not containing amino acids, 2% maltose, 2% lecithin emulsion, 0.1 M citric acid-sodium citrate buffer pH 4.0, 2% agarose, and 10 mM CaCl₂.

PDA solid medium: Purchased from BD Company, Cat. No. BD 213400.

YPD liquid medium: 1% yeast extract, 2% peptone, and 2% glucose.

MGYS solid medium: 1.34% yeast nitrogen base (YNB) containing ammonium sulfate and not containing amino acids, 1% glycerol, 1 M sorbitol, 4×10-5% D-biotin, and 2% agar.

BMM-soybean phospholipid screening medium: 1.34% yeast nitrogen base (YNB) containing ammonium sulfate and not containing amino acids, 4×10-5% D-biotin, 0.5% methanol (added after sterilization), 2% soybean phospholipid emulsion, 0.1 M citric acid-sodium citrate buffer pH 6.6, and 2% agar, to which 10 um of ZnSO4·7H2O was added.
-- 2% soybean phospholipid emulsion: 2 g of soybean phospholipid, 100 ml of H₂O, homogenized with a high-speed homogenizer at 8000 rpm for 1 min.

BMGY liquid medium: 1% yeast extract, 2% peptone, 1.34% yeast nitrogen base (YNB) containing ammonium sulfate and not containing amino acids, 1% glycerol, 4×10-5% D-biotin, and 0.1 M potassium dihydrogen phosphate-dipotassium hydrogen phosphate buffer pH 6.0.

BMMY liquid medium: 1% yeast extract, 2% peptone, 1.34% yeast nitrogen base (YNB) containing ammonium sulfate and not containing amino acids, 0.3% ZnSO4·7H2O, 0.5% methanol (added after sterilization), 4×10-5% D-biotin (added after sterilization), and 0.1 M citric acid-sodium citrate buffer pH 6.6.
Modified Bradford protein concentration assay kit (purchased from Sangon Biotech (Shanghai) Co., Ltd.)
PCR enzyme: PrimeSTAR^{®} HS DNA Polymerase (purchased from Takara Bioengineering (Dalian) Co., Ltd.)
NEFA kit (purchased from Wako Pure Chemical Industries, Ltd., Japan)

Phosphatidic acid was purchased from Aladdin reagent.

### Example 1 Construction of Mutant D94N and Determination of Enzyme Activity

### 1. Construction of plasmids

1.1 The construction of the plasmid pAOP-Eno was as described above.

### 1.2 Construction of pAOP-AN02-LPL and pAOP-D94N plasmids

The mature peptide of AN02-LPL was selected, as shown below in SEQ ID NO: 1:

AN02-LPL (SEQ ID NO: 1)

DISSTVLDNIDLFAQYSAAAYCSSNIESTGTTLTCDVGNCPLVEAAGATTIDEFDD
TSSYGDPTGFIAVDPTNELIVLSFRGSSDLSNWIADLDFGLTSVSSICDGCEMHKGFYEA
WEVIADTITSKVEAAVSSYPDYTLVFTGHSYGAALAAVAATVLRNAGYTLDLYNFGQP
RIGNLALADYITGQNMGSNYRVTHTDDIVPKLPPELLGYHHFSPEYWITSGNDVTVTTSD
VTEVVGVDSTAGNDGTLLDSTTAHRWYTIYISECS

Aspartic acid (D) at position 94 of AN02-LPL (SEQ ID NO: 1) was mutated to asparagine (N), and the mutated polypeptide was designated D94N.

The amino acid sequence (SEQ ID NO: 2) of the mature peptide of mutant D94N

DISSTVLDNIDLFAQYSAAAYCSSNIESTGTTLTCDVGNCPLVEAAGATTIDEFDD
TSSYGDPTGFIAVDPTNELIVLSFRGSSDLSNWIADLNFGLTSVSSICDGCEMHKGFYEA
WEVIADTITSKVEAAVSSYPDYTLVFTGHSYGAALAAVAATVLRNAGYTLDLYNFGQP
RIGNLALADYITGQNMGSNYRVTHTDDIVPKLPPELLGYHHFSPEYWITSGNDVTVTTSD
VTEVVGVDSTAGNDGTLLDSTTAHRWYTIYISECS

The AN02-LPL and D94N were sent to Genewiz (Suzhou) Biotechnology Co., Ltd. for gene sequence synthesis, and cloning into the pAOP-Eno plasmid afforded the pAOP-AN02-LPL plasmid and the pAOP-D94N plasmid.

### 2. Obtaining of Aspergillus niger mutant strains and determination of enzyme activity

The pAOP-AN02-LPL plasmid and the pAOP-D94N plasmid were separately transformed into an Aspergillus niger AN19 strain, and the resulting strains were designated 19-AN02-LPL and 19-D94N, respectively. The specific transformation method was as follows:
(1) Spores of the Aspergillus niger AN19 strain cultured in the PDA solid medium (purchased from BD, Cat. No. BD 213400) were removed by washing using a spore washing solution. The removed spores were vortexed for 1 min and filtered using Mircloth to prepare a uniform spore suspension.
(2) The suspension was inoculated at 1×10⁷ spores into a fermentation medium (2% glucose, 6% maltose, 7% sodium citrate, 1.5% ammonium sulfate, 4% Tryptic soy broth, 0.1% sodium dihydrogen phosphate, 0.1% magnesium sulfate, 0.07% Tween 80, and trace elements (KI 0.83 g/L, H₃BO₃ 6.2 g/L, MnSO₄.4H₂O 22.3 g/L, ZnSO₄.7H₂O 8.6 g/L, Na₂MoO₄.2H₂O 0.25 g/L, CuSO₄.5H₂O 0.025 g/L, and CoCl₂.6H₂ 0.025 g/L, added in a ratio of 1/1000; and FeSO₄.7H₂O 2.78 g/L and Na₂.EDTA 3.73 g/L, added in a ratio of 1/100)), and was cultured at 28°C and 200 rpm for 42 h to 48 h. The grown hyphae were collected by filtration using sterilized Mircloth (purchased from Milliproe). The collected hyphae were washed with a sterilized osmotic pressure stabilizer (0.6 mol/L MgSO4) three times, and dried under pressure.
(3) The hyphae were transferred to a 100 mL Erlenmeyer flask. Every 0.8 g of the hyphae was resuspended in 10 mL of an enzymolysis solution (1% cellulase (purchased from Sigma, Cat. No. C1184-25KU), 1% lywallzyme (purchased from Sigema, Cat. No. L1412-25G), 0.1% snailase (purchased from Sangon, Cat. No. A600870-0005)) and dispersed; 30°C, 60 rpm, 60 min to 90 min (observed once every 10 min after 30 min). The enzyme-digested hyphae were filtered using Mircloth, and then rinsed with 0.6 mol/L MgSO4, and the filtrate was collected. The filtrate was centrifuged at 4°C and 1000 g for 10 min, and the supernatant was discarded to obtain a protoplast pellet. The protoplast pellet was resuspended in 5 mL of a pre-cooled 1.0 mol/L sorbitol solution. The suspension was centrifuged at 800 g and 4°C for 10 min, and the supernatant was discarded. The protoplasts were resuspended in 1 mL of pre-cooled 1.0 mol/L sorbitol and placed in an ice bath for later use.
(4) The protoplasts were taken and centrifuged, and were adjusted to 1×10⁷ protoplasts/mL using a pre-cooled STC (1.0 M sorbitol, 50 mM CaCl₂, 50 mM Tris-HCl, pH = 7.5) solution to obtain a protoplast suspension. To 200 µL of the protoplast suspension were added 5 µg DNA (the pAOP-AN02-LPL plasmid or the pAOP-D94N plasmid) and 50 µL of a PTC (40% w/v PEG 4000, 50 mM CaCl₂, 50 mM Tris-HCl, pH = 7.5) solution. The mixture was gently tapped to mix homogeneously, and was placed on an ice bath for 30 min. 0.2 mL of the PTC solution was added and mixed homogeneously, and then 0.8 mL of the PTC solution was added and mixed homogeneously, at room temperature for 30 min. The above mixed solution was added to 5 ml of a regeneration medium (0.2% (w/v) KH₂PO₄, 0.1% (w/v) KCl, 0.05% (w/v) MgSO₄·7H₂O, 0.005% (w/v) FeSO₄·7H₂O, 1 M sucrose, 10 mM acetamide, 20 mM cesium chloride, and 0.6 w/v% agar), and mixed homogeneously. The resulting mixed solution was plated onto a regeneration plate containing the regeneration medium (except for containing 1.5% agar, the other components were the same as above), and was cultured at 28°C for three days or more.
(5) The clones grown out on the regeneration plate in the above step (4) were picked and added to the screening plate. 100 transformants were screened out from each strain. From these transformants, 10 transformants forming a large white precipitation circle on the screening plate were selected to perform shake flask fermentation, and the enzyme activities were compared. The specific process was as follows:
   (6) The 10 positive clones obtained from the screening were each coated onto 3% (w/v) PDA solid medium (purchased from BD Company, Cat. No. BD 213400), and cultured at 28°C for around 3 days until a large number of spores formed. The spores were removed by washing, vortexed for 1 min, and filtered using Mircloth to prepare a uniform spore suspension. The suspension was inoculated at 1×10⁷ spores into a fermentation medium (2% (w/v) glucose, 10% (w/v) maltose, 7% (w/v) sodium citrate, 1.5% (w/v) ammonium sulfate, 4% Tryptic soy broth, 0.1% (w/v) sodium dihydrogen phosphate, 0.1% (w/v) magnesium sulfate, 0.07% (w/v) Tween 80, and trace elements) and cultured at 28°C and 200 rpm for 8 days, and the enzyme activity was determined. The transformant having the highest enzyme activity was selected for enzyme activity comparison.
   (7) Determination of the enzyme activities of the fermentation broths of 19-AN02-LPL and 19-D94N strains before storage

The fermentation broths of 19-AN02-LPL and 19-D94N strains were taken and enzyme activity was determined using soybean lecithin as a substrate. The method was as follows: 10 ul of the above fermentation broth was added to 90 ul of a substrate solution, and a reaction was allowed to proceed at 50°C for 10 min. The reaction was inactivated at 95°C for 5 min, and centrifuged at 7000 rpm for 5 min. 1 ul of the supernatant was taken, 80 ul of reagent A in the NEFA kit was added, and a reaction was allowed to proceed at 37°C for 10 min. Then 160 ul of reagent B was added, and a reaction was allowed to proceed for 10 min. The absorbance at 550 nm was determined to calculate the enzyme activity.

The composition of the substrate solution (9 mL) consisted of 5 ml of 4% w/v soybean lecithin, 1 mL of 20% (v/v) Triton X-100, 2.5 mL of 0.1 M citric acid-sodium citrate buffer (pH = 4.3), and 0.5 mL of water.

The results showed that the enzyme activities of the fermentation broths of the 19-AN02-LPL strain and the 19-D94N strain were 47688 U/ml and 45457 U/ml, respectively.

### (8) Determination of the enzyme activities of the fermentation broths of the 19-AN02-LPL and 19-D94N strains after storage

The fermentation broths of the 19-AN02-LPL and 19-D94N strains were stored at 4°C for two weeks, and the enzyme activities were determined using the same method as in the above step (7).

The results showed that after two weeks of storage, the enzyme activity of the fermentation broth of the 19-AN02-LPL strain decreased from 47688 U/ml to 37261 U/ml; whereas the enzyme activity of the fermentation broth of the 19-D94N strain only decreased from 45457 U/ml to 43442 U/ml. Thus, the loss of enzyme activity was reduced by 77% for D94N compared to AN02-LPL. This facilitates stable storage of the enzyme and prolongs the period of use of the enzyme.

### Example 2: Construction of Mutant HLRT and Specific Enzyme Activity against Phosphatidic Acid

The mature peptide of AN02-LPL was selected, as shown in SEQ ID NO: 1.

Lysine at position 111 was mutated to histidine, tyrosine at position 147 was mutated to leucine, serine at position 84 was mutated to arginine, and aspartic acid at position 85 was mutated to threonine.

The amino acid sequence of the new mutant HLRT obtained was as follows:

DISSTVLDNIDLFAQYSAAAYCSSNIESTGTTLTCDVGNCPLVEAAGATTIDEFDD
TSSYGDPTGFIAVDPTNELIVLSFRGSRTLSNWIADLDFGLTSVSSICDGCEMHHGFYEA
WEVIADTITSKVEAAVSSYPDYTLVFTGHSLGAALAAVAATVLRNAGYTLDLYNFGQP
RIGNLALADYITGQNMGSNYRVTHTDDIVPKLPPELLGYHHFSPEYWITSGNDVTVTTSD
VTEVVGVDSTAGNDGTLLDSTTAHRWYTIYISECS (SEQ ID NO: 3)

The amino acid sequences of AN02-LPL and HLRT were sent to Genewiz (Suzhou) Biotechnology Co., Ltd. for gene sequence synthesis, and cloning into the pAO815 vector afforded P-AN02-LPL and P-HLRT plasmids. After linearization with BglII, the vector was transformed into competent cells of a Pichia pastoris GS115 strain by electroporation. The transformants were inoculated onto the MGYS solid medium plate and cultured at 30°C for 3 days, thus obtaining Pichia pastoris transformants of pic-AN02-LPL and pic-HLRT. The monoclones on the plate were picked and added to the BMM-soybean phospholipid screening medium plate. Clones having a large white precipitate circle were selected and designated pic-AN02-LPL and pic-HLRT.

The pic-AN02-LPL and pic-HLRT were taken, first activated in the YPD liquid medium, then inoculated in the BMGY liquid medium, and cultured overnight at 30°C under shaking at 220 rpm. The respective cultures were transferred to the BMMY medium, the initial OD600 being 6.

First, induction was performed using 2% methanol. 1% methanol was supplemented separately after 24 h and 32 h, and 1% methanol was supplemented separately after 48 h and 56 h. Sampling was performed at 72 h.

The respective fermentation broths obtained were concentrated 40-fold by performing desalting by ultrafiltration using an ultrafiltration tube having a molecular weight cut-off of 10 kDa. The respective treated samples were added to a buffer (20 mM citric acid-sodium citrate buffer (pH 5.5)). The respective enzyme solutions obtained were designated AN02-LPL and HLRT.

The enzyme activity was determined using phosphatidic acid as a substrate. The method was as follows:
9 ml of substrate:5 ml of 1% phosphatidic acid, 1 ml of 20% Triton X-100, 2.5 ml of 0.1 M citric acid-sodium citrate buffer (pH = 4.3; 4.8; 5.5), and 0.5 ml of water.

10 ul of diluted enzyme solution + 90 ul of substrate were reacted at 50°C for 10 min. The reaction was inactivated at 95°C for 5 min, and centrifuged at 7000 rpm for 5 min. 1 ul of the supernatant was taken, 80 ul of reagent A in the NEFA kit was added, and a reaction was allowed to proceed at 37°C for 10 min. Then 160 ul of reagent B was added, and a reaction was allowed to proceed for 10 min. The absorbance at 550 nm was determined to calculate the enzyme activity.

The protein concentrations of the respective fermentation broths were determined using the modified Bradford protein concentration assay kit, and the specific enzyme activities of AN02-LPL and HLRT against phosphatidic acid under different pH conditions were calculated.

The specific enzyme activity of the new mutant HLRT against phosphatidic acid increased from 347 U/mg of AN02-LPL to 835 U/mg at pH4.3; increased from 863 U/mg of AN02-LPL to 2903 U/mg at pH 4.8; and increased from 0 U/mg of AN02-LPL to 2527 U/mg at pH 5.5.

### Example 3: HLRT Degumming Test

Pre-degummed bean crude oil was taken and shaken uniformly, and heated under stirring, the temperature being maintained at 55°C. 50% citric acid was added to a final concentration of 650 ppm. Shearing was performed at 20,000 rpm for 1.5 min. A reaction was allowed to proceed under stirring at 55°C for 1 h. 30 ppm of HLRT, 30 ppm of AN02-LPL, and 60 ppm of AN02-LPL were mixed with 1.8 ml of water, respectively. The respective enzyme-water mixed solutions were added, and shearing was performed at 200,000 rpm for 1.5 min. After addition of the enzyme-water mixed solutions, the temperature was raised to 55°C, and timing was started. A reaction was allowed to proceed for 4 h, and after the reaction was completed, the temperature was raised to 85°C to inactivate the enzymes by at least maintaining for > 8 min. Centrifugation was performed to obtain degummed oils, and the degummed oils were sampled to perform a phosphorus content test. A phosphorus content of less than 10 ppm was considered up to standard.

| | Phosphorus content (ppm) |
|---|---|
| HLRT 30 ppm | 7.8 |
| AN02-LPL 30 ppm | 21.9 |
| AN02-LPL 60 ppm | 8.1 |

### Example 4: Construction of Mutant M1RT and Specific Enzyme Activity against Phosphatidic Acid

A mutant polypeptide M1 of AN02-LPL (the mutation sites were L86I, G187D, E209K, and A254D based on the mature peptide) was prepared. The sequence of M1 is as shown below:

DISSTVLDNIDLFAQYSAAAYCSSNIESTGTTLTCDVGNCPLVEAAGATTIDEFDD
TSSYGDPTGFIAVDPTNELIVLSFRGSSDISNWIADLDFGLTSVSSICDGCEMHKGFYEAW
EVIADTITSKVEAAVSSYPDYTLVFTGHSYGAALAAVAATVLRNAGYTLDLYNFGQPRI
GNLALADYITDQNMGSNYRVTHTDDIVPKLPPKLLGYHHFSPEYWITSGNDVTVTTSDV
TEVVGVDSTDGNDGTLLDSTTAHRWYTIYISECS (SEQ ID NO: 4)

Serine at position 84 of M1 was mutated to arginine, and aspartic acid at position 85 was mutated to threonine.

The amino acid sequence of the new mutant M1RT obtained is as follows:

DISSTVLDNIDLFAQYSAAAYCSSNIESTGTTLTCDVGNCPLVEAAGATTIDEFDD
TSSYGDPTGFIAVDPTNELIVLSFRGSRTISNWIADLDFGLTSVSSICDGCEMHKGFYEAW
EVIADTITSKVEAAVSSYPDYTLVFTGHSYGAALAAVAATVLRNAGYTLDLYNFGQPRI
GNLALADYITDQNMGSNYRVTHTDDIVPKLPPKLLGYHHFSPEYWITSGNDVTVTTSDV
TEVVGVDSTDGNDGTLLDSTTAHRWYTIYISECS (SEQ ID NO: 5)

The amino acid sequence of M1RT was sent to Genewiz (Suzhou) Biotechnology Co., Ltd. for gene sequence synthesis, and cloning into the pAO815 vector afforded a P-M1RT plasmid. After linearization with BglII, the vector was transformed into competent cells of a Pichia pastoris GS115 strain by electroporation. The transformants were inoculated onto the MGYS solid medium plate and cultured at 30°C for 3 days, thus obtaining Pichia pastoris transformants of pic-M1RT. The monoclones on the plate were picked and added to the BMM-soybean phospholipid screening medium plate. A clone having a large white precipitate circle was selected and designated pic-M1RT.

The pic-M1RT was taken, first activated in the YPD liquid medium, then inoculated in the BMGY liquid medium, and cultured overnight at 30°C under shaking at 220 rpm. The culture was transferred to the BMMY medium, the initial OD600 being 6.

First, induction was performed using 2% methanol. 1% methanol was supplemented separately after 24 h and 32 h, and 1% methanol was supplemented separately after 48 h and 56 h. Sampling was performed at 72 h.

The fermentation broth obtained was concentrated 40-fold by performing desalting by ultrafiltration using an ultrafiltration tube having a molecular weight cut-off of 10 kDa. The treated sample was added to a buffer (20 mM citric acid-sodium citrate buffer (pH 5.5)). The enzyme solution obtained was designated M1RT. The enzyme activity was determined using phosphatidic acid as a substrate. The method was as follows:
9 ml of substrate:5 ml of 1% phosphatidic acid, 1 ml of 20% Triton X-100, and 2.5 ml of 0.1 M citric acid-sodium citrate buffer (pH = 4.3; 4.8; 5.5).

10 ul of diluted enzyme solution + 90 ul of substrate were reacted at 50°C for 10 min. The reaction was inactivated at 95°C for 5 min, and centrifuged at 7000 rpm for 5 min. 1 ul of the supernatant was taken, 80 ul of reagent A in the NEFA kit was added, and a reaction was allowed to proceed at 37°C for 10 min. Then 160 ul of reagent B was added, and a reaction was allowed to proceed for 10 min. The absorbance at 550 nm was determined to calculate the enzyme activity.

The protein concentration of the fermentation broth was determined using the modified Bradford protein concentration assay kit, and the specific enzyme activities of M1RT against phosphatidic acid under different pH conditions were calculated.

The specific enzyme activity of the new mutant M1RT against phosphatidic acid increased from 347 U/mg of AN02-LPL to 1252 U/mg at pH 4.3; increased from 863 U/mg of AN02-LPL to 2956 U/mg at pH 4.8; and increased from 0 U/mg of AN02-LPL to 2972 U/mg at pH 5.5.

### Example 5: Construction of Mutant WTRT and Specific Enzyme Activity against Phosphatidic Acid

The mature peptide of AN02-LPL was selected, as shown in SEQ ID NO: 1.

Serine at position 84 was mutated to arginine and aspartic acid at position 85 was mutated to threonine. The amino acid sequence of the new mutant WTRT obtained is as follows:

DISSTVLDNIDLFAQYSAAAYCSSNIESTGTTLTCDVGNCPLVEAAGATTIDEFDD
TSSYGDPTGFIAVDPTNELIVLSFRGSRTLSNWIADLDFGLTSVSSICDGCEMHKGFYEA
WEVIADTITSKVEAAVSSYPDYTLVFTGHSYGAALAAVAATVLRNAGYTLDLYNFGQP
RIGNLALADYITGQNMGSNYRVTHTDDIVPKLPPELLGYHHFSPEYWITSGNDVTVTTSD
VTEVVGVDSTAGNDGTLLDSTTAHRWYTIYISECS (SEQ ID NO: 6)

The amino acid sequence of WTRT was sent to Genewiz (Suzhou) Biotechnology Co., Ltd. for gene sequence synthesis, and cloning into the pAOP-Eno vector afforded a pAOP-WTRT plasmid. An Aspergillus niger AN19 strain was transformed.

The transformation method was as follows:
Spores of the Aspergillus niger strain cultured in the PDA solid medium were removed by washing using a spore washing solution. The removed spores were vortexed for 1 min and filtered using Mircloth to prepare a uniform spore suspension. The suspension was inoculated at 1×10⁷ spores into a fermentation medium (2% glucose, 6% maltose, 7% sodium citrate, 1.5% ammonium sulfate, 4% Tryptic soy broth, 0.1% sodium dihydrogen phosphate, 0.1% magnesium sulfate, 0.07% Tween 80, and trace elements), and cultured at 28°C and 200 rpm for 42 h to 48 h. The grown hyphae were collected by filtration using sterilized Mircloth. The collected hyphae were washed with a sterilized osmotic pressure stabilizer (0.6 mol/L MgSO₄) three times, and dried under pressure. The hyphae were transferred to a 100 mL Erlenmeyer flask. Every 0.8 g of the hyphae was resuspended in 10 mL of an enzymolysis solution and dispersed; 30°C, 60 rpm, 60 min to 90 min (observed once every 10 min after 30 min). The protoplast mixed solution was filtered using Mircloth, and then rinsed with 0.6 mol/L MgSO₄, and the filtrate was collected. The filtrate was centrifuged at 4°C and 1000 g for 10 min, and the supernatant was discarded. The protoplast pellet was resuspended in 5 mL of a pre-cooled 1.0 mol/L sorbitol solution. The suspension was centrifuged at 800 g and 4°C for 10 min, and the supernatant was discarded. The protoplasts were resuspended in 1 mL of pre-cooled 1.0 mol/L sorbitol and placed in an ice bath for later use. The protoplasts were taken and centrifuged, and were adjusted to 1×10⁷ protoplasts/mL using a pre-cooled STC (1.0 M sorbitol, 50 mM CaCl₂, 50 mM Tris-HCl, pH = 7.5) solution. To 200 µL of the protoplast suspension were added 5 µg of DNA and 50 µL of the PTC (40% PEG 4000, 50 mM CaCl2, 50 mM Tris-HCl, pH = 7.5) solution. The mixture was gently tapped to mix homogeneously, and placed on an ice bath for 30 min. 0.2 mL of the PTC solution was added and mixed homogeneously, and then 0.8 mL of the PTC solution was added and mixed homogeneously, at room temperature for 30 min. The above mixed solution was added to 5 ml of a regeneration medium (0.2% KH₂PO₄, 0.1% KCl, 0.05% MgSO₄·7H₂O, 0.005% FeSO₄·7H₂O, 1 M sucrose, 10 mM acetamide, 20 mM cesium chloride, and 0.6% agar), and mixed homogeneously. The resulting mixed solution was plated onto the regeneration medium (the other components were the same as above, 1.5% agar), and was cultured at 28°C for three days or more.

The positive clones grown out on the regeneration medium were coated onto the PDA solid medium, and were cultured at 28°C for around 3 days until a large number of spores formed. The spores were removed by washing, vortexed for 1 min, and filtered using Mircloth to prepare a uniform spore suspension. The suspension was inoculated at 1×10⁷ spores into a fermentation medium (2% glucose, 10% maltose, 7% sodium citrate, 1.5% ammonium sulfate, 4% Tryptic soy broth, 0.1% sodium dihydrogen phosphate, 0.1% magnesium sulfate, 0.07% Tween 80, and trace elements), and cultured at 28°C and 200 rpm for 8 days for determination of enzyme activity.

The enzyme activity was determined using phosphatidic acid as a substrate. The method was the same as in Example 4. The specific enzyme activity of the new mutant WTRT against phosphatidic acid increased from 347 U/mg of AN02-LPL to 984 U/mg at pH 4.3; increased from 863 U/mg of AN02-LPL to 2003 U/mg at pH 4.8; and increased from 0 U/mg of AN02-LPL to 1342 U/mg at pH 5.5.

### Example 6: Construction of Mutant WTRT-Y

The mature peptide of AN02-LPL was selected, as shown in SEQ ID NO: 1.

Serine at position 84 was mutated to arginine, aspartic acid at position 85 was mutated to threonine, and aspartic acid at position 94 was mutated to tyrosine. The amino acid sequence of the new mutant WTRT-Y obtained is as follows:

DISSTVLDNIDLFAQYSAAAYCSSNIESTGTTLTCDVGNCPLVEAAGATTIDEFDD
TSSYGDPTGFIAVDPTNELIVLSFRGSRTLSNWIADLYFGLTSVSSICDGCEMHKGFYEA
WEVIADTITSKVEAAVSSYPDYTLVFTGHSYGAALAAVAATVLRNAGYTLDLYNFGQP
RIGNLALADYITGQNMGSNYRVTHTDDIVPKLPPELLGYHHFSPEYWITSGNDVTVTTSD
VTEVVGVDSTAGNDGTLLDSTTAHRWYTIYISECS (SEQ ID NO: 7).

The amino acid sequence of WTRT-Y was sent to Genewiz (Suzhou) Biotechnology Co., Ltd. for gene sequence synthesis, and cloning into the pAOP-Eno vector afforded a pAOP-WTRT-Y plasmid.

### Example 7: Comparison of the Enzyme Activity of Mutant WTRT-Y with That of AN02-LPL and Mutant WTRT

The expression vectors pAOP-AN02-LPL (see Example 1 for the construction method), pAOP-WTRT (see Example 5 for the construction method), and pAOP-WTRT-Y were transformed into Aspergillus niger AN19E (see patent application CN202111598184.2). The transformation method was as follows:
Fresh Aspergillus niger AN19E spores were removed by washing using a spore washing solution. The spores were prepared as a spore suspension by Miracloth filtration, and adjusted to 1×10⁷/mL. 1 mL of the spore suspension was inoculated into a hyphae medium (2% tryptone, 1% yeast extract, 2% glucose, and 0.3% uracil), and cultured at 28°C and 180 rpm for 40 hours. The grown hyphae were collected by filtration with sterilized Miracloth.

The collected hyphae were washed with a sterilized osmotic pressure stabilizer (0.6 M MgSO₄, 10mM NaH₂PO₄, pH=5.8) three times, and dried under pressure. The hyphae were transferred to a 100 mL Erlenmeyer flask. Every 0.8 g of the hyphae was resuspended in 20 mL of an enzymolysis solution (the enzymolysis solution was 1% lyase, 1% cellulase, and 0.1% snailase prepared using the osmotic pressure stabilizer, and was sterilized by filtration using a 0.22 µm microporous filter membrane). Enzymolysis was performed at 30°C and 90 rpm for 60 min to 90 min. The protoplast mixed solution obtained from the enzymolysis was filtered using Miracloth. The filtrate was collected and centrifuged at 4°C and 1000 g for 10 min. The protoplast pellet was resuspended in 5 mL of pre-cooled 1.0 mol/L sorbitol solution and centrifuged at 800 g and 4°C for 10 min, and the supernatant was discarded. The protoplasts were then adjusted to 1×10⁷ protoplasts/mL using a pre-cooled STC solution (1.0 M sorbitol, 50 mM CaCl₂, 50 mM Tris-HCl, pH=7.5), and the protoplast suspension was placed on an ice bath for later use.

To 200 µL of the protoplast suspension, 10 µL of the expression vector at a concentration of 1 µg/µL was added, and then 50 µL of the PTC solution (40% PEG 4000, 50 mM CaCl₂, 50 mM Tris-HCl, pH=7.5) was further added. The mixture was mixed homogeneously and placed on an ice bath for 30 min. 0.2 mL of the PTC solution was added and mixed homogeneously, and then 0.8 mL of the PTC solution was added and mixed homogeneously. The resulting mixed solution was maintained at room temperature for 30 min.

The above mixed solution was plated onto a regeneration medium (1% glucose, 0.6% NaNO3, 0.15% KH2PO4, 0.05% KCl, 0.05% MgSO4, 0.001% FeSO4, 1 M sucrose, and 2% agar powder), and was cultured at 28°C for 7 days until colonies grew out.

The colonies grown out on the plate were transferred to the Aspergillus niger screening plate and cultured at 28°C for 3 days to obtain Aspergillus niger expression strains of LPL, i.e., pAOP-AN02-LPL, pAOP-WTRT, and pAOP-WTRT-Y.

The strains were subjected to shake flask fermentation. The fermentation medium (2% glucose, 15% maltose, 7% sodium citrate, 1.5% ammonium sulfate, 4% TSB, 0.1% sodium dihydrogen phosphate, 0.1% magnesium sulfate, 0.07% Tween 80, and trace elements) was autoclaved at 115°C for 15 min. The fermentation conditions were 28°C, 220 rpm, 10 d, and an inoculum dose of 1×10⁷ spores/50 mL.

After the shake flask fermentation was completed, the enzyme activity was determined. The fermentation broths of pAOP-AN02-LPL, pAOP-WTRT, and pAOP-WTRT-Y were diluted to an appropriate protein concentration, and the enzyme activity was determined using phosphatidic acid as a substrate. The method was the same as in Example 4.

The results showed that the new mutant WTRT-Y could still maintain a high specific enzyme activity against phosphatidic acid, and the specific enzyme activity increased from 347 U/mg of AN02-LPL to 961 U/mg at pH 4.3; increased from 863 U/mg of AN02-LPL to 1803 U/mg at pH 4.8; and increased from 0 U/mg of AN02-LPL to 1212 U/mg at pH 5.5. The enzyme activities were substantially equivalent to those of the mutant WTRT (984 U/mg at pH 4.3, 2003 U/mg at pH 4.8, and 1342 U/mg at pH 5.5).

### Example 8: Evaluation of the Anti-degradation Stability of the Mutants WTRT and WTRT-Y

The supernatants of the pAOP-WTRT and pAOP-WTRT-Y fermentation enzyme solutions were microfiltered and then stored at 4°C and -20°C respectively for two weeks. The changes in residual lipase enzyme activity were investigated to evaluate the anti-degradation stability of the mutant genes.

The method for determining the enzyme activity was the same as in Example 4.

The results showed that the mutant WTRT showed a decrease in enzyme activity from 29921 U/ml to 20076 U/ml after two weeks of storage at 4 degrees, while the mutant WTRT-Y showed an increase in protein stability in that the enzyme activity thereof only decreased from 28765 U/ml to 25984 U/ml after two weeks of storage at 4 degrees. The loss decreased by 68% compared to that of WTRT.

### Example 9: Evaluation of the Strain Stability of the Mutants WTRT and WTRT-Y

The pAOP-WTRT and pAOP-WTRT-Y strains were subcultured two times on PDA solid medium plates, and were subjected to shake flask fermentation to investigate the changes in enzyme activity after subculture.

The results are shown in FIG. 1. As shown in FIG. 1, the mutant WTRT, after being subcultured two times, had an enzyme activity of only 37.5% of the non-subcultured strain, whereas the new mutant WTRT-Y, after being subcultured two times, had an enzyme activity consistent with that of the non-subcultured strain, without the phenomenon of instability.

## Claims

1. A mutant lysophospholipase, having one or more mutations in the amino acid sequence thereof relative to a wild-type lysophospholipase and having increased specific activity or increased stability.

2. The lysophospholipase according to claim 1, wherein the amino acid sequence thereof has a mutation or mutations at one or more positions selected from positions 84, 85, and 94 corresponding to SEQ ID NO: 1.

3. The lysophospholipase according to claim 2, wherein the amino acid sequence thereof has a mutation at position 94 corresponding to SEQ ID NO: 1, and preferably, the mutation is mutation of aspartic acid to asparagine.

4. The lysophospholipase according to claim 2, wherein the amino acid sequence thereof has mutations at positions 84 and 85 corresponding to SEQ ID NO: 1, and preferably, the mutations are mutation of serine at position 84 to arginine and mutation of aspartic acid at position 85 to threonine.

5. The lysophospholipase according to claim 4, further comprising an additional mutation or additional mutations at one or more positions selected from positions 111, 147, 86, 187, 209, and 254 corresponding to SEQ ID NO: 1, preferably, the additional mutations are mutations at positions 111 and 147, or mutations at positions 86, 187, 209, and 254, and more preferably, the additional mutations are mutation of lysine at position 111 to histidine and mutation of tyrosine at position 147 to leucine; or mutation of leucine at position 86 to isoleucine, mutation of glycine at position 187 to aspartic acid, mutation of glutamic acid at position 209 to lysine, and mutation of alanine at position 254 to aspartic acid.

6. The lysophospholipase according to claim 2, wherein the amino acid sequence thereof has mutations at position 84, 85, and 94 corresponding to SEQ ID NO: 1; preferably, the mutations are mutation of serine at position 84 to arginine, mutation of aspartic acid at position 85 to threonine, and mutation of aspartic acid at position 94 to tyrosine.

7. The lysophospholipase according to claim 1, comprising the amino acid sequence shown in SEQ ID NO: 2, 3, 5, 6, or 7.

8. A nucleic acid molecule, selected from:
(a) a nucleotide sequence encoding the lysophospholipase according to any one of claims 1-7; and
(b) a nucleotide sequence complementary to the nucleotide sequence according to (a).

9. A vector, comprising the nucleic acid molecule according to claim 8.

10. A host cell, comprising the nucleic acid molecule according to claim 8, or the vector according to claim 9; preferably, the host cell is selected from a bacterial cell, a fungal cell, a mammalian cell, an insect cell, and a plant cell, and more preferably, the host cell is a fungal cell, more preferably a Pichia pastoris cell or an Aspergillus niger cell.

11. A method for producing a lysophospholipase, comprising expressing a nucleic acid molecule encoding the lysophospholipase according to any one of claims 1-7 in a host cell, and recovering a resulting polypeptide.

12. A composition, comprising a fermentation broth, a fermentation supernatant, and/or a fermentation concentrate of the lysophospholipase according to any one of claims 1-7 or the host cell according to claim 10.

13. A fermentation broth, a fermentation supernatant, or a fermentation concentrate of the host cell according to claim 10.

14. A use of the lysophospholipase according to any one of claims 1-7, the composition according to claim 12, or the fermentation broth, the fermentation supernatant, and/or the fermentation concentrate according to claim 13 in oil degumming.
